# EUROPEAN PATENT APPLICATION

(11) **EP 1 927 348 A1**
(43) Date of publication of application: **04.06.2008**
(21) Application number: 06301170.4
(22) Date of filing: 22.11.2006
(51) Int. Cl.: A61K 31/00, A61K 31/195, A61K 45/06, A61P 25/08, A61P 27/02

(54) **Anticonvulsive pharmaceutical compositions**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Catherine, Alain

(57) **Abstract**

The present invention pertains to pharmaceutical compositions comprising, as the active ingredients, a combination of vigabatrin and of at least one glutamate receptor activating substance.

## Description

### FIELD OF THE INVENTION

The present invention relates to anticonvulsive pharmaceutical compositions having reduced undesirable effects comprising the anticonvulsive agent vigabatrin in combination with at least one glutamate receptor activating substance.

### BACKGROUND OF THE INVENTION

Epilepsy is a general term describing a group of central nervous system disorders that are characterized by recurrent seizures that are the outward manifestation of excessive and/or hyper-synchronous abnormal electrical activity of neurons of the cerebral cortex and other regions of the brain. This abnormal electrical activity can be manifested as motor, convulsion, sensory, autonomic, or psychic symptoms.

Epilepsy is one of the more common neurological disorders and affects millions of people worldwide, and over 2.5 million individuals in the United States.

It is believed that the characteristic seizures of epilepsy are caused by the disordered, synchronous, and rhythmic firing of brain neurons. The neurons can fire at up to four times their normal rate. As a result, epileptic seizures are an overstimulation of the normal neuronal processes that control brain function.

Even though existing antiepileptic drugs can render 80% of newly diagnosed patients seizure free, a significant number of patients have chronic intractable epilepsy causing disability with considerable socioeconomic implications.

Anti-epileptic drugs are available for treating epilepsies, but these agents have a number of shortcomings. For instance, the agents are often poorly soluble in aqueous and biological fluids or are extremely hygroscopic. Of even greater importance is that patients often become refractory to a drug over time. In addition, many anti-epileptic agents cause unwanted side effects, neurotoxicities, and drug interactions. Even while being treated with one or a combination of the anti-epileptic drugs currently in clinical use, 30% of epileptic patients still experience seizures. As more anti-epileptic drugs are developed, the clinician will have expanded pharmaceutical options when designing an effective treatment protocol for each patient.

Vigabatrin, which was developed as an inhibitor of gamma-aminobutyric acid transaminase, was one of the most promising novel anticonvulsant active ingredients. However, vigabatrin was shown to induce highly severe undesirable effects, such as an irreversible constriction of the visual field. The constriction of the visual field induced by vigabatrin is asymptomatic when it is restricted to the nasal quadrant, until it extends to more central areas. Furthermore, visual defects induced by vigabatrin are not limited to the constriction of the visual field but also includes dysfunction of central vision with a reduction of visual acuity, a loss of color discrimination and of contrast sensitivity. An arrest of a therapeutical treatment with vigabatrin allows a stabilization of the visual loss but very rarely induces any recovery.

However, because epileptic seizures are always very handicapping and may be lethal, vigabatrin is still prescribed.

There is thus a need in the art for improved anticonvulsive, including anti-epileptic, pharmaceutical compositions comprising vigabatrin, which would be endowed with reduced or no undesirable effects.

### SUMMARY OF THE INVENTION

It is provided according to the present invention novel anticonvulsive pharmaceutical compositions comprising vigabatrin, which compositions possess reduced undesirable effects, as compared with the vigabatrin-based pharmaceutical compositions known in the art.

One object of the present invention consists of a pharmaceutical composition comprising, as the active ingredients, a combination of vigabatrin and of at least one substance inducing an activation of a glutamate receptor within the retina tissue.

This invention also pertains to a method for treating convulsive disorders, including epilepsy, comprising a step of administering, to a patient in need thereof, a combination of vigabatrin and of at least one substance inducing an activation of a glutamate receptor within the retinal tissue.

According to the present invention, substances inducing an activation of glutamate receptor within the retina tissue encompass glutamate, glutamate derivatives, glutamate metabolites and glutamate analogues, inhibitors of glutamate reuptake, agonists of glutamate receptors and substances increasing glutamate release.

### DETAILED DESCRIPTION OF THE INVENTION

Surprisingly, it has been found according to the invention that the progressive and irreversible loss of visual acuity of patients treated with vigabatrin is associated with a local decrease in glutamate concentration within the retinal tissue, the said decrease in glutamate concentration inducing retinal plasticity with formation of ectopic synapses and induction of photoreceptor dysplasia.

More precisely, the inventors have shown that all ON bipolar cells (bipolar cells depolarizing at the light onset) and horizontal cells undergo plasticity following treatment with vigabatrin at both rod and cone photoreceptor terminals. Rod photoreceptors were also found to withdraw their terminals toward their cell bodies. Photoreceptor withdrawal and dendritic growth of postsynaptic neurons are associated with a loss of synaptic transmission between photoreceptors and their postsynaptic neurons. The plasticity of retinal neurons at the photoreceptor synapses that is shown herein demonstrated a defect in synaptic transmission from photoreceptors to postsynaptic neurons during the vigabatrin treatment. The results obtained by the inventors are thus fully consistent with a reduction in glutamate and glutamine concentration caused by vigabatrin because photoreceptors are glutamatergic neurons. This reduction of glutamate concentration may contribute significantly to the formation of ectopic synapses, with additional mechanisms that may contribute to photoreceptor dysplasia. These results are fully corroborated by the additional inventors findings showing that the vigabatrin-induced retinal plasticity is prevented by maintaining the vigabatrin-treated individuals in darkness, thus in conditions wherein a sufficient rate of glutamate release is preserved although significantly lower than in normal conditions. Glutamate release is indeed maximum in dark-adapted conditions and it is reduced by light in a linear function of the light intensity.

The inventors findings that vigabatrin induces a local decrease in the local glutamate release within the retinal tissue, which decrease is - at least partly- responsible from the deleterious side-effects of this anticonvulsant substance, have allowed them to design improved vigabatrin-based anticonvulsive methods and compositions with reduced side-effects, as compared to the methods and compositions including vigabatrin that are already known in the art, wherein vigabatrin is combined with at least one substance inducing an activation of glutamate receptors within the retinal tissue, which substance may also be termed herein a "glutamate receptor activating substance".

Vigabatrin consists of the International Common Name of 4-amino-5-hexenoic acid, which may also be termed 4-aminohex-5-enoic acid (IUPAC designation), which chemical formula is C₆H₁₁N0₂, and which has the CAS Registry accession number 60643-86-9.

An object of the present invention consists of an anticonvulsive pharmaceutical composition comprising, as the active ingredients, a combination of (i) 4-amino-5-hexenoic acid and (ii) at least one glutamate receptor activating substance.

By the expression "at least one substance", it is intended herein "one or more substance(s)".

As used herein, an anticonvulsive pharmaceutical composition consists of a pharmaceutical composition that is active for preventing and treating convulsive disorders. Convulsive disorders encompass epilepsy, tuberous sclerosis, as well as the convulsive disorders affecting patients undergoing a drug addiction, including a drug addiction to heroin or cocaine.

As used herein, glutamate receptor activating substances encompass all substances that, when administered by local route or alternatively by systemic route, cause an activation of glutamate receptors, which include (i) substances inducing an increase in glutamate concentration within the retina tissue, (ii) substances that inhibit reuptake of synaptically-released glutamate or that facilitate glutamate transporter-mediated glutamate release, (iii) substances that directly activate glutamate receptors, e.g. agonists of the glutamate receptors and (iv) substances that increase glutamate release by increasing glutamate uptake into synaptic vesicles or by facilitating synaptic vesicular release of glutamate.

Substances inducing an increase in glutamate concentration encompass those that cause an increase in the amount of glutamate that should normally be present in the local inter-synaptic local areas included in the retinal tissue, and more particularly in the local areas of the retinal tissue located between photoreceptor cells and postsynaptic neurons.

Thus, glutamate receptor activating substances encompass (i) glutamate, substances that are transformed into glutamate by chemical or enzymatic reaction, substances comprising glutamate and substances generating glutamate or glutamate-containing compounds after chemical or enzyme reaction, (ii) substances that inhibit glutamate reuptake or favor glutamate transporter-mediated release by glutamate-releasing cells and (iii) substances that activate the glutamate receptor by mimicking or enhancing the glutamate activity, (iii) substances that facilitate glutamate vesicular release.

In an anticonvulsive pharmaceutical composition according to the invention, vigabatrin is combined to any glutamate receptor activating substance(s). Notably, in an anticonvulsive pharmaceutical composition according to the invention, vigabatrin is combined with any one of those substances that are already known to exert a glutamate receptor activating activity.

In certain embodiments of an anticonvulsive pharmaceutical composition according to the invention, the glutamate receptor activating substance(s) is (are) selected from the group consisting of (i) glutamate, substances that are transformed into glutamate by chemical or enzymatic reaction, substances comprising glutamate and substances generating glutamate or glutamate-containing compounds after chemical or enzymatic reactions, (ii) substances that inhibit glutamate reuptake by glutamate-releasing cells by inhibiting photoreceptor glutamate transporters, by stimulating glutamate release through the transporters, (iii) substances that activate the glutamate receptor by mimicking or increasing the glutamate activity and (iv) substances increasing glutamate vesicular release.

An anticonvulsive pharmaceutical composition according to the invention may comprise more than one glutamate receptor activating substance, so as to further reduce the deleterious effect of vigabatrin on the retinal tissue.

Thus, in certain embodiments of an anticonvulsive pharmaceutical composition as described herein, the said composition may comprise, additionally to vigabatrin, 2, 3, 4, 5, 6, 7, 8, 9 or 10 distinct glutamate receptor activating substances, especially among those belonging to the various classes of glutamate receptor activating substances that are listed above.

In certain embodiments, each of the more than one glutamate receptor activating substance that is comprised in an anticonvulsive pharmaceutical composition according to the invention belongs to a specific class of glutamate receptor activating substances, which class may be distinct from the classes to which belong the at least one other glutamate receptor activating substances combined therewith.

However, in most embodiments, an anticonvulsive pharmaceutical composition according to the present invention comprises, in combination to vigabatrin, 1, 2 or 3 distinct glutamate receptor activating substances, wherein, optionally, each of the glutamate receptor activating substance belongs to a specific class of glutamate receptor activating substances among those listed above, which class is distinct from the class(es) to which belong the other(s) glutamate receptor activating substance(s) combined therewith.

As it is already mentioned above, certain embodiments of an anticonvulsive pharmaceutical composition according to the invention comprise, as the glutamate receptor activating substance(s), a first class of substance(s) including glutamate, substances that are transformed into glutamate by chemical or enzymatic reactions, substances comprising glutamate and substances generating glutamate or glutamate-containing compounds after chemical or enzymatic reactions. These glutamate receptor activating substances thus encompass glutamate *per se,* as well as glutamate derivatives, glutamate metabolites, glutamate-generating substances, glutamate analogues, including mixtures thereof.

This first class of glutamate receptor activating substances encompasses glutamate and glutamine, as well as salts thereof and mixtures thereof.

This first class of substances also encompasses alpha-ketoglutaric acid (AKG) as well as AKG-containing substances like ornitine-AKG, arginine-AKG, glutamine-AKG, glutamate-AKG and leucine-AKG, as well as salts of AKG, including salts of AKG with amino acids. This first class of substances also include salts of alpha-ketoglutaric acid (AKG) selected from the group consisting of mono-metal salt of AKG, di-metal salt of AKG, mono-metal salt of glutamate and di-metal salt of glutamate. These include for example calcium or natrium salts of AKG, as well as calcium or natrium salts of glutamate.

This first class of substances further encompasses glutamate-containing dipeptides and glutamate-containing oligopeptides like, illustratively, L-alanyl-L-glutamate and L-glycyl-L-glutamate. These also include glutamate polymers.

The second class of glutamate receptor activating substances comprises inhibitors of glutamate reuptake or stimulators of glutamate transporter-mediated release. The said inhibitors of glutamate reuptake encompass any one of those inhibitors that are already known in the art, including inhibitors of glutamate transporter, like, illustratively, threo-3hydroxy-DL-aspartic acid (THA), (2*S*)-trans-pyrrolidine-2,4-dicarboxylic acid (PDC), aminocaproic acid, and (2*S*,3*S*)-3-{3-[4-(Trifluoromethyl)benzoylamino]benzyloxy}aspartate or inhibitors of cystine-glutamate antiporters like (*S*)-4-carboxyphenylglycine.

The third class of glutamate receptor activating substances encompasses glutamate receptor agonists, i.e. substances that increase glutamate receptor activity, especially in the retinal tissue. The glutamate receptor agonists may be selected from the group consisting of indirect (allosteric) glutamate receptor agonists, direct glutamate receptor agonists, and, in some instances, partial glutamate receptor agonists.

As used herein, the term "glutamate receptor" encompasses various glutamate receptors, including both ionotropic receptors (iGluR) and metabotropic receptors (mGluR). Ionotropic receptors intended herein include N-methyl-D-aspartic acid (NMDA) type, kainic acid (KA) type and alpha-amino-3-hydroxy-5-methyl-4-isoxazolepropionic acid (AMPA) type, the two latter types of receptors being also currently termed as non-NMDA-type glutamate receptors.

The third class of glutamate receptor activating substances, consisting of glutamate receptor agonists, include LY354740 which is a conformationally constrained analog of glutamate consisting of a mGluR2/3 agonist, LY544344 which is a prodrug of LY354740, L-HCSA, L-homocysteine sulfinic acid; L-HCA, L-homocysteic acid L-CSA, L-cysteine sulfinic acid; L-CA, L-cysteic acid which are all agonist at all groups of mGluR receptors, (S)-3,5-dihydroxyphenylglycine (DHPG), which is a group I mGluR receptor agonist, 2R,4R-APDC, which is a group II mGluR agonist, ACPT-1 and 2-amino-4-(3-hydroxy-5-methylisoxazol-4-yl)butyric acid which are two group III mGluR agonists (S)-2-(4-fluoro-phenyl)-1-(toluene-4-sulfonyl)-pyrrolidine (Ro67-7476); , diphenylacetyl-carbamicacid ethyl ester (Ro 01-6128) and (9H-xanthene-9-carbonyl)-carbamic acid butyl ester (Ro 67-4853), which are three mGluR1 positive allosteric modulators, 3,3V-difluorobenzaldazine (DFB) and the second by N-{4-chloro-2-[(1,3-dioxo-1,3-dihydro-2Hisoindol-2-yl)methyl]phenyl}-2-hydroxybenzamide, which are two mGluR-5 positive allosteric modulators, N-(4-(2-methoxyphenoxy)phenyl)-N-(2,2,2-trifluoroethylsulfonyl)prid-3-ylmethylamine (LY487379), which is a mGluR2 positive allosteric modulator, (_)-N-Phenyl-7-(hydroximino)cyclopropa[b]chromen-1a-carboxamide ((_)-PHCCC), which is a positive allosteric modulator of mGluR4, (1S,3R,4S)-1-aminocyclopentane-1,2,4-tricarboxylic acid and 2-amino-4-(3-hydroxy-5-methylisoxazol-4-yl)butyric acid which are agonists of mGluR4, L-(+)-2-Amino-4-phosphonobutyric acid and 2-amino-4-(3-hydroxy-5-methylisoxazol-4-yl)butyric acid, which are two mGluR6 agonists, (R,S)-3,4-DCPG, which is a mGluR8 receptor agonist, N-methyl-D-aspartic acid which is a NMDA receptor agonist, d-Cycloserine, which is a NMDA receptor agonist, glycine which is a NMDA receptor agonist, spermidine which is a NMDA receptor agonist, milacemide which is a NMDA receptor agonist, cis-ACPD which is a NMDA receptor agonist, homoquinolinic acid which is a NMDA receptor agonist, quisqualate which is a mGluR agonist, (*S*)-3,5-dihydroxyphenylglycine [(*S*)-DHPG which is a mGluR agonist, (_)-2-Oxa-4-aminobicyclo[3.1.0.]hexane-4,6-dicarboxylate(LY379268) which is a mGluR agonist, (2*S*,1'*S*,2'*S*)-2-(carboxycyclopropyl)glycine (L-CCG-I) which is an agonist of the metabotropic glutamate receptor, alpha-amino-3-hydroxy-5-methyl-4-isoxazolepropionic acid (AMPA), polyamines which are AMPA receptor agonists, S-(-)-5-fluorowillardine which is an AMPA receptor agonist, (RS)-Willardine which is an AMPA receptor agonist, Ampakines which are AMPA receptor agonists, kainic acid (KA), domic acid which is a KA receptor agonist and SYM 2081 which is a KA receptor agonist.

Another non-NMDA type glutamate receptor agonist dysiherbaine may also be used as a third class glutamate receptor activating substance, the said substance being disclosed in the US Patent N° 6,147,230.

Also, other NMDA receptor agonists such as (2S,1'R,2'R,3'R)-2-(2,3-dicarboxycyclopropyl)-glycine (DCG-I) and (2S,1'S,2'S,3'S)-2-(2,3-dicarboxycyclopropyl)glycine (DCG-II) may also be used as a third class glutamate receptor activating substances, the said substances being disclosed in the US Patent N° 5,334,757, as well as (2S,3R,4S)-carboxycyclopropylglycine as disclosed in the US Patent N° 4,959,493.

The fourth class of glutamate receptor activating substances encompasses stimulators of synaptic release, i.e. substances that increase glutamate release, especially in photoreceptors. The said stimulators of glutamate release encompass any one of those stimulators that are already known in the art. The said stimulators of glutamate release may be selected from (a) agonists or antagonists of presynaptic receptors regulating calcium channels, (b) compounds inducing vesicular release of glutamate and (c) molecules inducing a depolarization of photoreceptors (i.e. photoreceptor depolarizing compounds).

The fourth class of glutamate receptor activating substances, consisting of stimulators of glutamate release, includes (R,S)-alpha-methylserine-O-phosphate (MSOP), an antagonist of group III mGluR receptors, theophylline (aminophylline), which is an Adenosine receptor antagonists (S)PHPNECA, LUF5835 and LUF5845, which are Adenosine receptor A2 antagonists, Rp-cAMPS, which is an inhibitor of protein kinase A (PKA), Sildenafil and vardenafil, two inhibitors of the phodiesterase 6 (PDE6).

In certain embodiments of an anticonvulsive pharmaceutical composition according to the invention, the said pharmaceutical composition further comprises at least one substance having an anti-ischemic effect. Any one of the substances that are already known to exert an effect against ischemia, and especially an effect against retinal ischemia, are encompassed herein.

As used herein, "retinal ischemia" encompasses generally any disorder involving an hypoxic condition of the retinal tissue, especially any disorder that reduces availability of blood, oxygen or other nutrients to the retinal tissue and which can result in retinal tissue disorganization and retinal cell death. Thus, retinal ischemia encompasses hypoxic conditions of the retinal tissue, notably those that are caused by a reduction of the arterial blood flow or the venous blood flow to, or in, the retina, as well as those resulting from a dysfunction of the retinal pigment epithelium in transferring glucose and oxygen to photoreceptors.

In those embodiments of an anticonvulsive pharmaceutical composition according to the invention, the said substance having an anti-ischemic effect is preferably selected from the group consisting of an antioxidant substance, a free radical scavenger substance, a statin, an ACE inhibitor, an AT-1 antagonist, a calcium channel inhibitor, a sodium channel blocker agent, a potassium channel activator agent, a beta-adrenergic blocking agent, an anesthetics substance, an anticonvulsive agent, a hormone, a vasodilatator agent, an α-receptor antagonist, a xanthine oxidase inhibitor, a cyclooxygenase inhibitor, a protease inhibitor, an immunosuppressant agent and a mitochondrial ATP sensitive potassium opener agent.

Thus, in certain embodiments of an anticonvulsive pharmaceutical composition that comprise, in addition to vigabatrin and one or more glutamate receptor activating substance(s), also at least one anti-ischemic substance, and illustratively 2, 3, 4, 5, 6, 7, 8, 9 or 10 distinct anti-ischemic substances, especially among those belonging to the various classes of anti-ischemic substances that are listed above.

In some of these embodiments, each of the more than one anti-ischemic substance that is comprised in an anticonvulsive pharmaceutical composition according to the invention belongs to a specific class of anti-ischemic substances, which class is distinct from the classes to which belong the at least one other anti-ischemic substance combined therewith.

However, in most of these specific embodiments of an anticonvulsive pharmaceutical composition according to the present invention, the said composition comprises, in combination to vigabatrin and the at least one glutamate receptor activating substance, also 1, 2 or 3 distinct anti-ischemic substances, wherein, optionally, each of the anti-ischemic substance belongs to a specific class of anti-ischemic substances among those listed above, which class is distinct from the class(es) to which belong the other(s) anti-ischemic substance(s) combined therewith.

Illustratively, anti-ischemic substances may consist of antioxidant substances, such as antioxidant substances selected from the group consisting of glutathion, N-acetylcysteine, alpha-lipoic acid, resveratrol (CAS registry n° 501-36-0), ramelteon ((S)-N-[2-(1,6,7,8-tetrahydro-2H-indeno-[5,4-b] furan-8-yl)ethyl]propionamide ; CAS Registry n° 196597-26-9), a retinoid compound, an antioxidant vitamin, co-enzyme Q-10, beta carotene, uric acid, L-2-oxothiazolidine-4-carboxylic acid and melatonin.

The antioxidant vitamin may be selected from the group consisting of α-tocopherol, vitamin B, vitamin C, vitamin D, vitamin E, vitamin K, and salts thereof.

Other antioxidant substances may consist of antioxidant compounds selected from the group consisting of a flavonoid, a polyphenol, a phytooestrogen or an extract from Ginkgo biloba.

Further antioxidant substances may consist of antioxidant compounds selected from the group consisting of a SOD-like substance and a catalase-like substance.

Still further antioxidant substances may consist of free radical scavenger substances selected from the group consisting of tirilazad, ebselen, ederavone and melatonin.

### Pharmaceutical compositions and methods.

Generally, a pharmaceutical composition according to the invention comprises the combination of vigabatrin with at least one glutamate receptor activating substance and further also one or more physiologically acceptable excipients.

In specific embodiments of pharmaceutical compositions according to the invention, the said compositions further comprise one or more anti-ischemic substances, as it is described above.

Vigabatrin, the at least one glutamate receptor activating substance, and optionally the at least one anti-ischemic substance, are comprised in an anticonvulsive pharmaceutical composition in a "therapeutically effective amount", that is in an amount sufficient for the combination of active ingredients to exert the expected anticonvulsive effect while inducing no deleterious side effect, or side effects which are reduced as compared with the deleterious side effects induced by pharmaceutical compositions comprising vigabatrin without any glutamate receptor activating substance nor any anti-ischemic substance.

Generally speaking, a "therapeutically effective amount", or "effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent; i.e., carrier, or additive.

The present invention also concerns methods for preventing or treating convulsive disorders comprising a step of administering (i) a combination of vigabatrin and of at least one glutamate receptor activating substance or (ii) a pharmaceutical composition as defined above, to an individual in need thereof.

The individuals in need of such treatments encompass those, either adult or child patients, which are susceptible to convulsive disorders, especially epilepsy.

Thus, another object of the present invention consists of a method for preventing or treating convulsive disorders of a patient comprising a step of administering to a patient in need thereof a combination of (i) 4-amino-5-hexenoic acid and (ii) at least one glutamate receptor activating substance.

In some embodiments, the method above comprises a step of administering to a patient in need thereof a combination of (i) 4-amino-5-hexenoic acid, (ii) at least one glutamate receptor activating substance and (iii) at least one anti-ischemic substance.

In certain embodiments, the said method comprises a step of administering to a patient in need thereof a pharmaceutical composition that is described in the present specification.

By "physiologically acceptable excipient or carrier" is meant solid or liquid filler, diluent or substance which may be safely used in systemic or topical administration. Depending on the particular route of administration, a variety of pharmaceutically acceptable carriers well known in the art include solid or liquid fillers, diluents, hydrotropes, surface active agents, and encapsulating substances.

Pharmaceutically acceptable carriers for systemic administration that may be incorporated in the composition of the invention include sugar, starches, cellulose, vegetable oils, buffers, polyols and alginic acid. Specific pharmaceutically acceptable carriers are described in the following documents, all incorporated herein by reference: U.S. Pat. No. 4,401,663, Buckwalter et al. issued August 30, 1983; European Patent Application No. 089710, LaHann et al. published Sept. 28, 1983; and European Patent Application No. 0068592, Buckwalter et al. published Jan. 5, 1983. Preferred carriers for parenteral administration include propylene glycol, pyrrolidone, ethyl oleate, aqueous ethanol, and combinations thereof.

Representative carriers include acacia, agar, alginates, hydroxyalkylcellulose, hydroxypropyl methylcellulose, carboxymethylcellulose, carboxymethylcellulose sodium, carrageenan, powdered cellulose, guar gum, cholesterol, gelatin, gum agar, gum arabic, gum karaya, gum ghatti, locust bean gum, octoxynol 9, oleyl alcohol, pectin, poly(acrylic acid) and its homologs, polyethylene glycol, polyvinyl alcohol, polyacrylamide, sodium lauryl sulfate, poly(ethylene oxide), polyvinylpyrrolidone, glycol monostearate, propylene glycol monostearate, xanthan gum, tragacanth, sorbitan esters, stearyl alcohol, starch and its modifications. Suitable ranges vary from about 0.5% to about 1%.

For formulating a pharmaceutical composition according to the invention, the one skilled in the art will advantageously refer to the last edition of the European pharmacopoeia or of the United States pharmacopoeia.

Preferably, the one skilled in the art will refer to the fifth edition "2005" of the European Pharmacopoeia, or also to the edition USP 28-NF23 of the United States Pharmacopoeia.

The weight amount of the combination of active ingredients that is contained in each dose of the pharmaceutical composition of the invention will depend on the molecular weight of said therapeutically active compound as well as on the weight amount that is effective in inhibiting or blocking the convulsive disorder. Effective amounts of vigabatrin that are needed for preventing or treating convulsive disorders are well known from the one skilled in the art.

For determining the appropriate amount of the glutamate receptor activating substance(s) to be combined with vigabatrin, in a dose of a pharmaceutical composition of the invention, the one skilled in the art may advantageously refer to the effective amounts that are already known or determined in the art for the anti-ischemic substance(s) that is (are) comprised therein.

Also, in certain embodiments, the appropriate amount of the anti-ischemic substance(s) to be combined with vigabatrin and the glutamate receptor activating substance(s), in a dose of a pharmaceutical composition of the invention, the one skilled in the art may advantageously refer to the effective amounts that are already known or determined in the art for the anti-ischemic substance(s) that is (are) comprised therein.

The present invention is further illustrated by the examples below.

### EXAMPLES

### A. Materials and Methods of the examples

### A.1. Animal treatment

BALB/c mice (16 treated, 10 controls) were purchased from Janvier (Le Genest-St-Isle, France) at six weeks. VGB dissolved in 0.9% NaCl at 100mg/ml was intraperitoneally injected daily for 30 days at a final concentration of 250mg/kg as described for animal treatment of epilepsy (Andre V, Ferrandon A, Marescaux C, Nehlig A. Vigabatrin protects against hippocampal damage but is not antiepileptogenic in the lithium-pilocarpine model of temporal lobe epilepsy. Epilepsy Res. 2001;47:99-117). This dose was slightly higher than that prescribed to adult patient (1-6mg/kg), children (100mg/kg), or infants (250mg/kg) (Bialer M, Johannessen SI, Kupferberg HJ et al. Progress report on new antiepileptic drugs: a summary of the Fifth Eilat Conference (EILAT V). Epilepsy Res. 2001;43:11-58).

### A.2. Histology

Eye cups were fixed overnight at 4°C in 4% (wt/vol) paraformaldehyde in phosphate buffered saline (PBS) 0.01 M, pH 7.4. After cryoprotection in PBS containing successively 10%, 20% and 30% sucrose at 4°C, the tissue was embedded in OCT (Labonord, Villeneuve d'Ascq, France). Vertical sections (8-10µm thickness) were permeabilized for 5 minutes in PBS containing 0.1% Triton X-100 (Sigma, St. Louis, MO), rinsed, and incubated in PBS containing 1% bovine serum albumin (Eurobio, Les-Ulis, France), 0.1% Tween 20 (Sigma), and 0.1% sodium azide (Merck, Fontenay-Sous-Bois, France) for 2 hours at room temperature. The primary antibody added to the solution was incubated for 2 hours at room temperature. The polycolonal antibodies were directed against the protein kinase C alpha (PKCα) (1:2000, Sigma), VGluT1 (1:2000, Chemicon), Goα (K-20; 1:100, Santa Cruz), calbindin D-28K (1:500, Chemicon), mouse cone arrestin/ Luminaire junior (LUMIj) (1:20,000, ²⁷). The monoclonal antibodies were directed against Goα (1:2000, Chemicon), bassoon (1:100, StressGen) and calbindin D-28K (1:500, Sigma). After rinses, sections were incubated with the secondary antibody, goat anti-rabbit IgG or rabbit anti-mouse IgG conjugated to either Alexa TM594 or Alexa TM488 (1:500, Molecular Probes, Eugene, OR) for 2 hours. The dye, diamidiphenyl-indole (DAPI) was added to the last incubated solutions. Sections were rinsed, mounted with Fluorsave reagent (Calbiochem, San Diego, CA) and viewed with a Leica microscope (LEICA DM 5000B) equipped with a Ropper scientific camera (Photometrics cool SNAP TM FX).

### B. Results of the Examples

### Example 1 : retinal changes in Vigabatrin-treated mice

In rats, the major retinal changes consisted in a retinal architecture disorganization, a major glial reaction, cone damages and photoreceptor apoptosis. Initially, we examined these previously observed retinal changes in VGB-treated mice. Areas with a highly disorganized outer nuclear layer (ONL) were also observed in VGB-treated albino mice but not in all animals (5 of 16 treated mice). The results show that photoreceptor layer can become disorganized in VGB-treated mice, as well as in VGB-treated rats.

To examine cone photoreceptors, these cells were immunolabelled with the specific mouse cone arrestin antibody (Zhu X, Li A, Brown B et al. Mouse cone arrestin expression pattern: light induced translocation in cone photoreceptors. Mol Vis. 2002;8:462-471). The results show the intense labelling of cone outer segments and of their terminals with the cone arrestin antibody. In VGB-treated mice, cone outer segments had disappeared, whereas their terminals remained intensely stained and some cell bodies became positive. These observations indicated the presence of cone damage in VGB-treated mice as in treated rats.

When the retinal sections of control albino mice were immunolabelled against GFAP, glial Müller cells were already intensely stained and this GFAP staining was not increased by the VGB-treatment. These observations indicated that retinal damages had similarities with those described in VGB-treated rats but with a reduced level of retinal gliosis.

To examine the reaction of inner retinal, ON bipolar cells were stained with the antibody directed against the protein Goα. In control animals, ON bipolar cells exhibited cell bodies with very short dendrites extending to the outer plexiform layer (OPL). By contrast, in VGB-treated animals, ON bipolar cells extended their dendrites deep into the ONL.

To determine if both rod and cone ON bipolar cells were growing dendrites into the ONL, retinal sections were double immunolabelled with the antibody directed against the protein kinase Cα (PKCα), which identify selectively ON rod bipolar cells. The results show that rod ON bipolar cells exhibited dendrites extending into the ONL. When PKCa and Goα double immunolabelled retinal sections were carefully examined, some Goα-positive dendrites that extended to the outer nuclear layer were found to be PKCα negative indicating thereby that cone ON bipolar cells also extended their dendrites into the ONL. These observations demonstrated that both rod and cone ON bipolar cells underwent plasticity following the VGB treatment.

To investigate whether horizontal cells follow the same pattern of plasticity, these were stained with an antibody directed against calbindin. In control retina, the labelling showed horizontal cells with their dendrites ramifying into the OPL as well as some amacrine cell bodies. In VGB-treated animals, horizontal cells extended their dendrites deep into the ONL like ON bipolar cells. Results obtained with DAPI-stained photoreceptor nuclei showed that this neuronal plasticity was observed in areas with no disorganization of the ONL. In fact, growing dendrites of postsynaptic neurons were present along the complete retinal sections, suggesting that these features of plasticity occurred in postsynaptic neurons prior to the ONL disorganization.

Because bipolar cells and horizontal cells normally have their dendritic tips associated at photoreceptor invaginating synapses, double immunolabelling examined if they remain associated during their dendritic sprouting. Although Goα-positive bipolar cell dendrites were often associated with calbindin-positive horizontal cell dendrites, a few Goα-positive dendrites appeared isolated from calbindin-positive processes. These observations indicated that the dendritic growth in horizontal cells and ON bipolar cells could occur independently in VGB treated animals.

To determine whether the sprouting of postsynaptic neurons is related to changes in photoreceptor synaptic terminals, we used antibodies against bassoon, a protein of the photoreceptor synaptic ribbon, and against VGLUT1, a protein of photoreceptor synaptic vesicles. On retinal sections of control animals, bassoon immunolabelling showed a punctuate distribution restricted to the OPL.

In contrast, in VGB-treated animals, the punctuate labelling was observed to penetrate deep into the ONL. Similarly, the VGLUT1 immunolabeling was restricted to the OPL in control animals whereas it was observed deep into the ONL in VGB-treated animals. These results indicated that photoreceptor terminals were withdrawn into the ONL close to photoreceptor nuclei. In contrast, cone photoreceptor terminals appeared to remain in the OPL even in highly disorganized areas.

To confirm the absence of cone photoreceptor terminal withdrawal in areas with dendritic growth of postsynaptic neurons, retinal sections were immunolabelled with the cone arrestin antibody while ON bipolar cells were identified with the Goα-antibody. Although the OPL structure in VGB-treated mice was not as linear as in control animals, cone terminals remained in the OPL whereas Goα-positive dendrites of ON bipolar cells were already detected very deep into the ONL. These observations suggested that rod but not cone photoreceptor terminals were withdrawn toward their cell bodies.

To investigate whether withdrawing rod photoreceptor terminals keep contact with their postsynaptic neurons, photoreceptor synaptic terminals were immunolabelled with the VGLUT1 antibody while ON bipolar cells were identified with the Goα antibody. This double immunostaining showed that although rod photoreceptor terminals were often facing a sprouting bipolar cell dendrite, few VGLUT1-positive photoreceptor terminals were not associated to a growing bipolar cell dendrite. To further examine whether rod photoreceptor synaptic ribbons had retracted away from their postsynaptic neurons, photoreceptor synaptic ribbons were immunolabelled with the bassoon antibody while rod ON bipolar cells were stained with the PKCa antibody. Again, although most bassoon-positive synaptic ribbons were facing a sprouting rod ON bipolar cell dendrite, a few synaptic ribbons were not contacted by a rod bipolar cell dendrite. These observations indicated that photoreceptor withdrawal and dendritic growth of postsynaptic neurons were associated with a loss of synaptic transmission between photoreceptors and their postsynaptic neurons.

### Example 2 : Light dependence of the vigabatrin-elicited retinal toxicity

Albino rats only are sensitive to the VGB-elicited retinal toxicity (Butler WH, Ford GP, Newberne JW. A study of the effects of vigabatrin on the central nervous system and retina of Sprague Dawley and Lister-Hooded rats. Toxicol Pathol. 1987;15:143-148). Therefore, we tested whether VGB retinal damages were due to light because the retina of albino animals receives more than a log unit higher luminance higher than that of pigmented animals (Lyubarsky AL, Daniele LL, Pugh EN, Jr. From candelas to photoisomerizations in the mouse eye by rhodopsin bleaching in situ and the light-rearing dependence of the major components of the mouse ERG. Vision Res. 2004;44:3235-3251). When VBG-treated animals were maintained in a dark room throughout the treatment, bipolar cells were found not to grow dendrites in the ONL as in control animals. Furthermore, photoreceptors did not retract their terminals in the ONL. Other retinal damages like ONL disorganization were not observed in these VGB-treated animals maintained in darkness whereas animals under the 12h light/dark cycle showed the bipolar cell dendritic growth and the photoreceptor terminal withdrawal. These results indicated that maintaining the animals in darkness during VGB treatment can prevent the retinal toxicity.

For summarizing the results of the Examples herein, it has been shown that vigabatrin induced plasticity of postsynaptic neurons which is consistent with a vigabatrin-induced defect in photoreceptor synaptic transmission. In VGB-treated animals, plasticity in neurons postsynaptic to photoreceptors therefore suggests that photoreceptor synaptic transmission was impaired by the treatment. This VGB-induced photoreceptor impairment is further indicated by the rod terminal withdrawal as observed in both retinal detachment and in bassoon knockout mice, in which ectopic synapses are also forming in the ONL. Considering cones, the photoreceptor impairment is supported by the loss of their outer segments although their terminals did not appear to withdraw. Therefore, the plasticity of retinal neurons at the photoreceptor synapses demonstrates a defect in synaptic transmission from photoreceptors, the photoreceptors consisting of glutamatergic neurons. More precisely, photoreceptors are glutamatergic neurons and continuously release glutamate in the dark with a modulation of this release by light stimulation such that the glutamate concentration in the synaptic cleft is a linear function of light intensity. The results shown in the Examples herein strongly suggest that the observed defect in photoreceptor synaptic transmission is related to a retinal reduction in glutamate and glutamine concentrations in vigabatrin-treated animals. This decrease in glutamate in vigabatrin-treated animals helps explaining the formation of ectopic synapses in the ONL. This decrease in glutamate concentration could therefore contribute significantly to the formation of ectopic synapses, but with additional mechanisms that may contribute to photoreceptor dysplasia. The prevention of this retinal plasticity when maintaining the animal in darkness is consistent with this importance of the decrease in glutamate. Indeed, the rate of glutamate release is maximum in darkness under normal conditions. Therefore, during the VGB treatment, because retinal plasticity results from an insufficient glutamate release at photoreceptor terminals, maintaining animals in darkness may preserve a sufficient rate of glutamate release although significantly lower than in normal conditions. These observations induce that VGB retinal toxicity may be suppressed by locally increasing the glutamate concentration in the retina.

## Claims

1. An anticonvulsive pharmaceutical composition comprising, as the active ingredients, a combination of (i) 4-amino-5-hexenoic acid and (ii) at least one glutamate receptor activating substance.

2. The pharmaceutical composition according to claim 1, wherein the at least one glutamate receptor activating substance is selected from the group consisting of glutamate, glutamate derivatives, glutamate metabolites and glutamate analogues.

3. The pharmaceutical composition according to claim 2, wherein the at least one glutamate receptor activating substance is selected from the group consisting of alpha-ketoglutaric acid (AKG), ornitine-AKG, arginie-AKG, glutamine-AKG, glutamate-AKG, leucine-AKG and salts of AKG with amino acids.

4. The pharmaceutical composition according to claim 2, wherein the at least one glutamate receptor activating substance consists of a salt of alpha-ketoglutaric acid (AKG) selected from the group consisting of mono-metal salt of AKG, di-metal salt of AKG, mono-metal salt of glutamate and di-metal salt of glutamate.

5. The pharmaceutical composition according to claim 2, wherein the at least one glutamate receptor activating substance is selected from the group consisting of glutamate dipeptides and glutamate oligopeptides.

6. The pharmaceutical composition according to claim 1, wherein the at least one glutamate receptor activating substance consists of an inhibitor of glutamate reuptake or that facilitate glutamate transporter-mediated glutamate release.

7. The pharmaceutical composition according to claim 1, wherein the at least one glutamate receptor activating substance consists of a glutamate receptor agonist.

8. The pharmaceutical composition according to claim 1, wherein the at least one glutamate receptor activating substance consists of a substance increasing the synaptic vesicular release of glutamate.

9. The pharmaceutical composition according to claim 1 which further comprises at least one substance having an anti-ischemic effect.

10. A method for preventing or treating convulsive disorders of a patient comprising a step of administering to a patient in need thereof a composition comprising a combination of (i) 4-amino-5-hexenoic acid and (ii) at least one glutamate receptor activating substance.

11. The method according to claim 10, which comprises a step of administering to a patient in need thereof a pharmaceutical composition according to any one of claims 2 to 9.

12. The method according to claim 10, wherein the said convulsive disorder consists of epilepsy.
